# EUROPEAN PATENT SPECIFICATION

(11) **EP 1 883 447 B1**
(45) Date of publication and mention of the grant of the patent: **02.09.2009**
(21) Application number: 06728345.7
(22) Date of filing: 11.05.2006
(51) Int. Cl.: A61M 31/00, A61M 37/00

(54) **A HIGH VELOCITY LIQUID-GAS STREAM DEVICE FOR ADMINISTERING THERAPEUTIC SUBSTANCES**
HOCHGESCHWINDIGKEITSFLÜSSIGGASSTROMGERÄT FÜR DIE VERABREICHUNG THERAPEUTISCHER SUBSTANZEN
DISPOSITIF A COURANT DE GAZ-LIQUIDE GRANDE VITESSE POUR L'ADMINISTRATION DE SUBSTANCES THERAPEUTIQUES

(30) Priority: 16.05.2005 US 681022 P
(43) Date of publication of application: 06.02.2008
(73) Proprietor: Tavtech Ltd., 56000 Yehud (IL)
(72) Inventor: TAVGER, Michael, 12900 Katzrin (IL)
(74) Representative: Burrows, Anthony Gregory
(86) International application number: PCT/IL2006/000557
(87) International publication number: WO 2006/123326

(56) References cited:
- WO-A-99/37229
- WO-A-20/05065032
- US-A- 5 022 414
- US-B1- 6 306 119
- US-B1- 6 673 081

## Description

### FIELD OF THE INVENTION

The present invention relates, generally, to devices for administering therapeutic substances, and, more specifically, to devices for applying a high velocity therapeutic liquid-gas stream for administering such substances to the skin.

### BACKGROUND OF THE INVENTION

It is known in the art to provide apparatus for dermal abrasion and the cleansing of exposed in vivo tissue. There are a multiplicity of applications to both humans and animals during surgical procedures where the removal from the tissue surface of solid contaminants, such as fibers, dust, sand particles, and the like, as well as organic matter, such as puss, fats, and others, is necessary.

In addition, such cleansing is necessary in preparation prior to and/or subsequent to treatment such as applying therapeutic substances to the tissue. In dental conditions such as gingivitis which is caused by the long-term effects of plaque deposits, unremoved plaque mineralizes into a hard deposit called calculus (tartar) that becomes trapped at the base of the tooth which often becomes a host for bacteria. After descaling or scraping away the accumulated calculus, it is necessary to cleanse the area at the base of the tooth and the surrounding gum tissue, thereby removing calculus debris and the toxins produced by the bacteria.

When a fluid stream is employed to irrigate a tissue surface, a boundary layer is formed which is characterized by having a fluid velocity which decreases sharply adjacent to the flow surface, and which is virtually zero at the tissue surface. As a result, those particles which are smaller than the thickness of the boundary layer of the fluid stream are often difficult or impossible to remove thereby. The smallest particles located in the boundary layer exhibit a drag resistance of a magnitude sufficient for these particles to remain attached to the surface and to resist being swept away by the fluid stream, even if this has an overall very high velocity.

International Patent Application Publication Number WO-A-2005/065032, "A High Velocity Liquid-Gas Mist Tissue Abrasion Device" to the present inventor, provides a thorough overview of the prior art for tissue cleaning and abrasion. Disclosed therein is a device for tissue cleaning and abrasion employing a high-velocity liquid-gas streaming mist that produces a minimal to negligible thickness boundary layer. However, neither the device disclosed therein nor any of the prior art provides improved treatment by including the direct application of therapeutic substances to the tissue being treated.

US6673081 to Tavger teaches an apparatus for dermal abrasion. The apparatus includes *inter alia* a container for a sterile liquid and a fluid delivery head for forming a liquid mist, the mist being ejected from the head at velocities that effect abrasion. The single liquid container does not allow for varying the concentration(s) of any substance(s) in the liquid which forms the mist.

### SUMMARY OF THE INVENTION

The present invention aims to provide a system for improved treatment of tissue, in particular human scalp, by direct application thereto of desired therapeutic substances in the form of a stream of therapeutic droplets carried in a high velocity gas.

A method of administering a therapeutic substance to tissue is described. The method includes the steps of:
a) accelerating a flow of gas, which is one of a group including at least one of air, oxygen, nitrogen, and carbon dioxide, through at least one gas discharge nozzle so as to provide a gas discharge flow at an elevated velocity;
b) introducing into the elevated velocity gas discharge flow at least one flow of therapeutic liquid, which is one of the group consisting of: saline solution and a solution comprising saline solution and at least one additional therapeutic substance, which may be a substance selected from a group which consists of: a medication, a nutrient, and a moisturizer, through at least one liquid discharge nozzle, thereby to fragment the at least one flow of therapeutic liquid into a stream of therapeutic droplets, and to accelerate the stream to an accelerated velocity similar to the velocity of the gas discharge flow; and
c) applying the accelerated therapeutic droplet stream to a tissue mass desired for therapeutic treatment thereby.

Further in accordance with a preferred embodiment of the invention, the step of applying is applying the accelerated therapeutic droplet stream to a human scalp in which hair may be present, which is desired for therapeutic treatment by the accelerated therapeutic droplet stream. Additionally, the accelerated therapeutic droplet stream may be applied to the tissue mass topically or subcutaneously. The step of applying the accelerated therapeutic droplet stream to a tissue mass may include holding in one hand a device for applying the accelerated therapeutic droplet stream. Additionally, the step of applying the accelerated therapeutic droplet stream to a tissue mass further includes cleansing the tissue mass thereby to remove contaminants from the tissue mass and dispersing accumulated liquid from the tissue mass by the flow of high velocity gas.

In further accordance with a preferred embodiment of the invention, in the step of introducing, the at least one flow of therapeutic liquid is one flow of saline solution, and the step of introducing further includes the step of supplying, possibly at preselected times for preselected time intervals, to the flow of saline solution a predetermined flow of at least one additional therapeutic substance from the above-mentioned group, thereby producing a mixed flow of therapeutic liquid having a predetermined concentration of the at least one additional therapeutic substance.

In accordance with an alternative preferred embodiment of the invention, in the step of introducing, the at least one flow of therapeutic liquid is at least two flows of therapeutic liquids, wherein a first flow of therapeutic liquid is a flow of saline solution and at least one additional flow of therapeutic liquid, which is possibly introduced at preselected times for preselected time intervals, is a predetermined flow of at least one additional therapeutic substance from the above-mentioned group, thereby producing a stream of therapeutic droplets containing a predetermined concentration of the at least one additional therapeutic substance.

Additionally in accordance with a preferred embodiment of the invention, the step of accelerating a flow of gas includes accelerating the flow of gas to a velocity either in the range of sub-sonic to supersonic velocity or the range of sonic to supersonic velocity.

Further in accordance with a preferred embodiment of the invention, the step of introducing into the elevated velocity gas discharge flow at least one flow of therapeutic liquid, includes the flow of gas entering the at least one gas discharge nozzle being at a pressure of a first magnitude, and the at least one gas discharge nozzle being operative to cause a pressure drop in the gas flow therethrough such that the pressure of the gas discharged from the at least one gas discharge nozzle is of a second magnitude, wherein the first magnitude is at least twice the second magnitude, thereby causing a shock wave in the gas and the at least one flow of liquid downstream of the at least one gas discharge nozzle and the at least one liquid discharge nozzle so as to cause atomizing of the therapeutic liquid discharged from the at least one liquid discharge nozzle into a high velocity stream of therapeutic droplets, thereby forming a stream of therapeutic droplets suspended in the flow of discharged high velocity gas.

A device for administering a therapeutic substance to tissue is described. The device includes:
a) a gas inlet port connected to a pressurized gas source at a pressure in the range of about 275 to 1035 KPa (40 to 150 psi), and including at least one gas selected from: air, oxygen, carbon dioxide and nitrogen;
b) at least one therapeutic liquid inlet port, each connected to a pressurized therapeutic liquid source at a pressure in the range of about 7 to 35 KPa (1 to 5 psi), and wherein the therapeutic liquid is one of the group which consists of: saline solution and a solution comprising saline solution and at least one additional therapeutic substance which is a substance selected from a group which consists of: a medication, a nutrient, and a moisturizer; and operative to supply therapeutic liquid at preselected times for preselected intervals; and
c) a stream jet delivery nozzle arrangement including;
   i) at least one gas discharge nozzle arranged to receive a flow of pressurized gas from the gas inlet port and configured to accelerate the flow of gas so as to discharge it at an elevated velocity in the range of sub-sonic to supersonic velocity; and
   ii) at least one liquid discharge nozzle arranged to receive a flow of therapeutic liquid from the at least one therapeutic liquid inlet port and operative to discharge the flow of therapeutic liquid into the elevated velocity flow of gas, thereby to similarly accelerate the velocity of the discharged therapeutic liquid as a therapeutic stream of accelerated therapeutic droplets and to discharge the stream of accelerated therapeutic droplets towards a tissue mass desired for therapeutic treatment by the therapeutic droplets.

Further in accordance with a preferred embodiment of the invention, the tissue mass desired for therapeutic treatment is a human scalp in which hair may be present.

In accordance with an alternative preferred embodiment of the invention, the stream jet delivery nozzle arrangement may include at least two gas discharge nozzles and at least two liquid discharge nozzles.

Additionally, in accordance with a preferred embodiment of the invention, the at least one liquid discharge nozzle is disposed substantially concentric and within the at least one gas discharge nozzle and the at least one gas discharge nozzle is a device configured to have a converging portion, a throat portion and a diverging portion. Further, the device is configured to be used while being held in one hand.

Further in accordance with a preferred embodiment of the invention, the flow of gas entering the at least one gas discharge nozzle is at a pressure of a first magnitude, and the at least one gas discharge nozzle is operative to cause a pressure drop in the gas flow therethrough such that the pressure of the gas discharged from the at least one gas discharge nozzle is of a second magnitude, wherein the first magnitude is at least twice the second magnitude, so as to cause a shock wave in the gas and the at least one flow of liquid downstream of the at least one gas discharge nozzle and the at least one liquid discharge nozzle so as to cause atomizing of the therapeutic liquid discharged from the at least one liquid discharge nozzle into a high velocity stream of therapeutic droplets, thereby to form a stream of therapeutic droplets suspended in the flow of discharged high velocity gas.

There is provided, in accordance with a preferred embodiment of the invention, a system for administering a therapeutic substance to tissue, including:
a) a pressurized gas source;
b) at least one pressurized therapeutic liquid source; and
c) a device as described hereinabove.

### BRIEF DESCRIPTION OF THE DRAWINGS

The present invention will be more fully understood and its features and advantages will become apparent to those skilled in the art by reference to the ensuing description, taken in conjunction with the accompanying drawings, in which:
Figure 1 is a perspective view of a device for administering therapeutic substances to tissue, constructed and operative in accordance with a preferred embodiment of the present invention;
Figure 2 is a schematic side view of the device of Fig. 1;
Figure 3 and 4 are enlarged schematic and graphical representations, respectively, of a delivery nozzle arrangement of the device seen in Figures 1 and 2;
Figure 5 is a schematic view of a flow of stream droplets discharging from the delivery nozzle arrangement as seen in Figure 4 against a surface to which therapeutic substances are to be administered;
Figure 6 is a schematic view of a flow of stream droplets discharging from the delivery nozzle arrangement seen in Figure 4, into a periodontal pocket;
Figure 7 is a schematic view of a nozzle arrangement, constructed and operative in accordance with an alternative embodiment of the present invention, having multiple gas and liquid discharge nozzles; and
Figure 8 is a block diagram of a system for administering therapeutic substances to tissue, in accordance with preferred embodiments of the present invention.

### DETAILED DESCRIPTION OF THE INVENTION

The present invention relates to a device for administering therapeutic substances to tissue by directing thereat a liquid-gas stream of droplets consisting of one or more therapeutic liquids at a high velocity generally within the range of sub-sonic to super-sonic. To achieve this, gas is discharged from a converging-diverging gas nozzle configured to accelerate the flow of gas so as to discharge it at an elevated velocity. A low rate of flow of therapeutic liquid is discharged into the elevated velocity flow of gas, thereby to similarly accelerate the velocity of the discharged therapeutic liquid as a therapeutic stream of accelerated droplets. The volumetric rate of flow of therapeutic liquid from the device is relatively low, thereby essentially preventing the formation of a virtually stagnant liquid boundary layer on the surface of the tissue to which therapeutic substances are to be administered.

When the therapeutic liquid administered by the present invention is saline solution, the invention can first be employed to clean a tissue surface, as described in International Patent Application Publication Number WO-A-2005/065032, "A High Velocity Liquid-Gas Mist Tissue Abrasion Device" to the present inventor, prior to administering additional therapeutic substances, such as medications, nutrients, or moisturizers; or colorants, any of which may be in liquid or soluble powder form. This allows more efficient dosing of the subsequent therapeutic substances, since, as will be appreciated by persons skilled in the art, the substances removed by cleaning would be likely, if left in place, to impede application and/or absorption of the desired therapeutic substances to the tissue desired for therapeutic treatment thereby. Included in the present invention are fluid flow control mechanisms known in the art operative to introduce into the device of the present invention a mixed flow of saline solution and other therapeutic substances, which may be in liquid or emulsion form, of a desired concentration therein which can further be controlled to only produce the mixed flow at specified times and for specified intervals. The device of the present invention would then accordingly produce a mixed therapeutic stream as desired and needed. Thus, as described above a tissue surface could first be cleaned by saline solution and then dosed therapeutically by a medication solution when it is ready to optimally receive the dosage. In an alternative embodiment of the present invention, instead of one mixed flow as mentioned hereinabove, the present invention further includes fluid flow control mechanisms known in the art operative to produce a number of therapeutic liquid flows for discharge into the elevated velocity flow of gas, which also may be turned on and off at specified times and for specified intervals. This arrangement also produces a mixed therapeutic stream as desired and needed.

For example, the present invention can be used to treat a human scalp, even where hair is present. First, the device produces an accelerated saline stream used to clean the scalp of extraneous material, excess oils, and dead epidermal tissue such as is known to produce dandruff. Then, a moisturizing, nutrient, anti-dandruff, or anti-hair loss, or other desired therapeutic substance is included in the accelerated stream to apply the desired therapeutic treatment to the scalp.

It should further be noted that the present invention is capable of applying the therapeutic substance to the desired tissue both topically and subcutaneously. Investigations employing prototype versions of the present invention have shown that the accelerated therapeutic stream produced thereby will, for suitable droplet flow velocities and length of time of exposure of the tissue to the droplet flow, penetrate the tissue surface. This capacity of non-invasive subcutaneous treatment and dosage is a further advantage of the present invention.

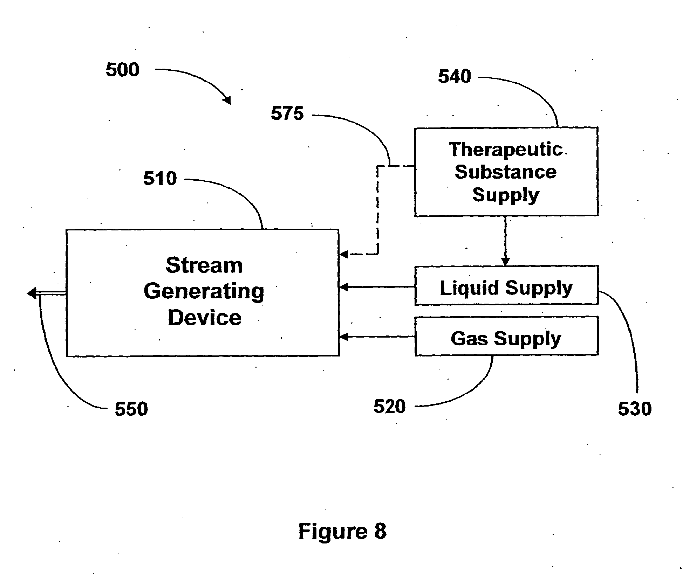

Referring now to Figure 8, there is shown a block diagram of a system, generally referred to as 500, for applying a high velocity liquid-gas therapeutic stream to tissue for therapeutic treatment thereof. System 500 employs a stream generating device 510 similar to that disclosed in International Patent Application Publication Number WO-A-2005/065032, "A High Velocity Liquid-Gas Mist Tissue Abrasion Device" to the present inventor, which is fed by high pressure gas supply 520 and high pressure liquid supply 530 and produces a high-velocity liquid-gas mist stream 550 suitable for tissue abrasion as described therein. The present invention further includes a supply of therapeutic substance 540, which may be in liquid or soluble powder form, that is introduced to liquid supply 530 so that the resulting high- velocity liquid-gas stream 550 includes the therapeutic substance which is thus applied to the tissue mass exposed to stream 550. Alternatively 575, the therapeutic substance is introduced directly into stream generating device 510 either in addition to (neutral) liquid supply 530 or when liquid supply 530 is turned off, thereby producing the desired high- velocity liquid-gas stream 550 which includes a predetermined concentration of the therapeutic substance.

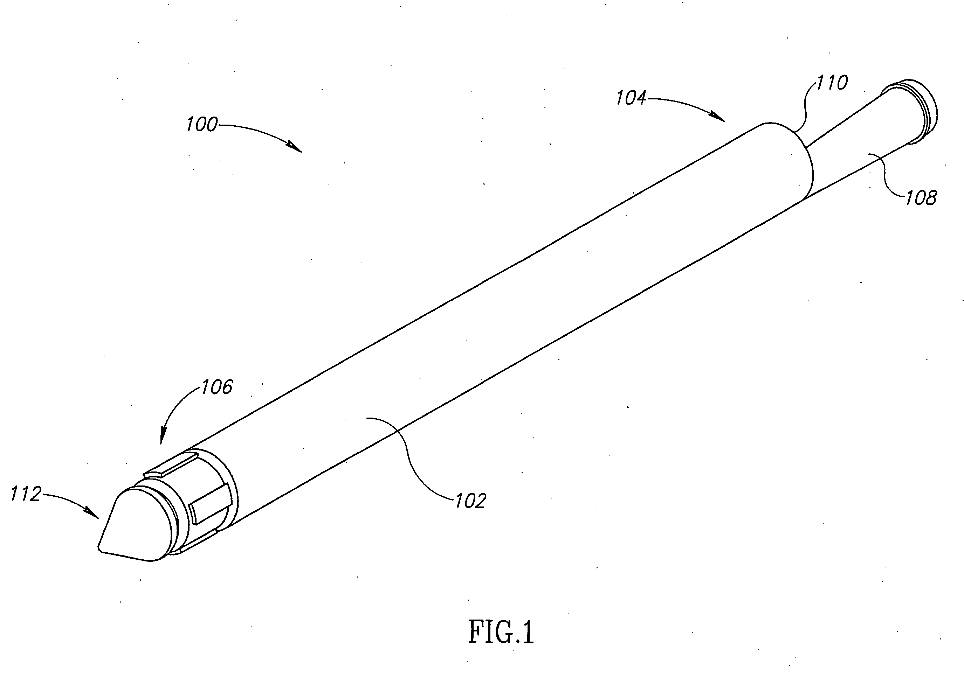

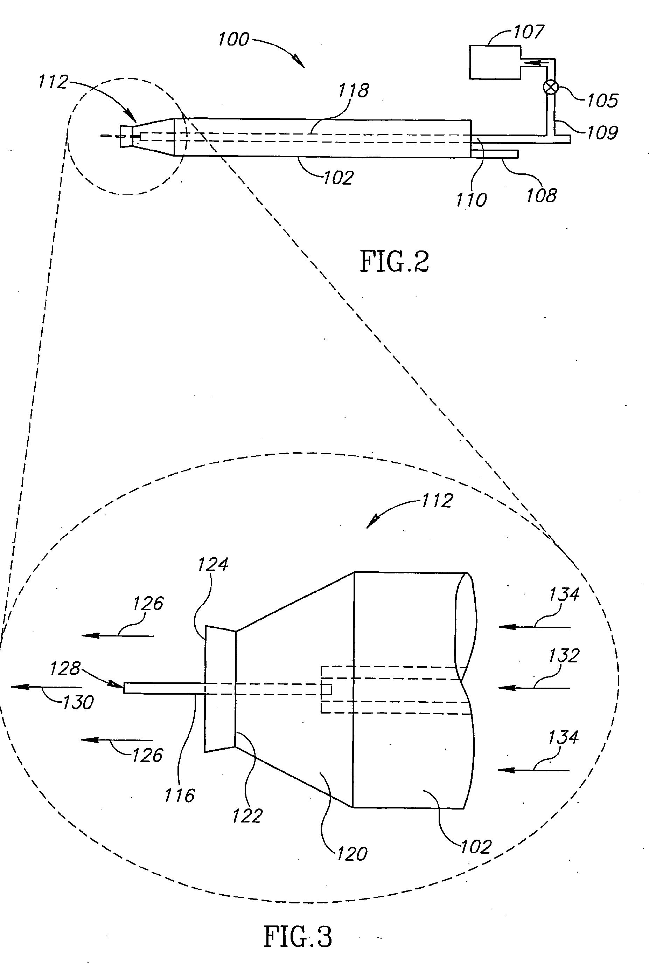

With reference to Figures 1 and 2, there is seen, according to a preferred embodiment of the present invention, a device referenced generally 100 for applying a high velocity liquid-gas therapeutic stream to tissue for therapeutic treatment thereof. Alternatively, the velocity of the stream may be regulated so as to merely provide cleansing of the tissue. Device 100 includes a housing portion referenced 102 having a generally tubular configuration, and having proximal and distal ends, referenced generally 104 and 106 respectively. A gas inlet port referenced 108 and a liquid inlet port referenced 110 are provided at proximal end 104, and a stream jet delivery nozzle arrangement referenced generally 112, is provided at distal end 106. In Figure 2, there is additionally shown, in schematic form, a therapeutic liquid inlet port 109 connecting pressurized therapeutic liquid source 107 liquid via flow control device 105 to liquid inlet port 110 to allow production of a mixed flow of therapeutic liquid. It should be noted that the present arrangement producing one mixed therapeutic liquid flow is only shown by way of example, and that multiple therapeutic liquid flows, as well as control of the time of application of different therapeutic liquid flows are also included in the present invention as discussed hereinabove.

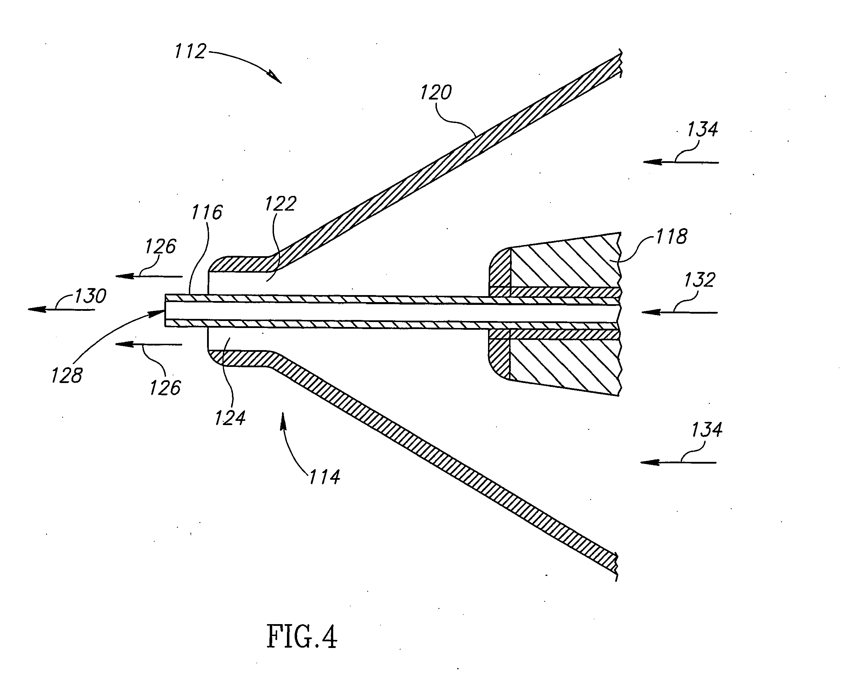

Referring now to Figures 3 and 4 in conjunction with Figure 2, there are seen schematic and graphical cross-sectional views of nozzle arrangement 112 of device 100. Nozzle arrangement 112 includes a gas discharge nozzle referenced generally 114 and, disposed generally concentrically there-within, is a liquid discharge nozzle referenced 116. Liquid inlet port 110 (Fig. 2) is connected in fluid flow communication with liquid discharge nozzle 116 by means of a liquid communication tube referenced 118, disposed generally concentrically within tubular housing portion 102 (Figs. 2 and 3).

Pressurized gas supplied from a pressurized gas source (not shown) enters device 100 through gas inlet port 108 (Fig. 2) and passes along and within tubular housing portion 102 as indicated by arrows 134, so as to discharge through gas discharge nozzle 114. Gas discharge nozzle 114 is generally configured having, in flow succession, a converging portion referenced 120, a throat portion referenced 122 and a diverging discharge portion referenced 124. The pressurized gas discharging from nozzle 114, as indicated by arrows 126, undergoes a rapid and substantial reduction in pressure to atmospheric pressure and a substantial acceleration to a high velocity, within the range of subsonic to supersonic velocity and specifically to a supersonic velocity. Gas discharge nozzle 114 is configured such that the discharging gas has an average cone angle of less than 10 degrees; that is, providing a substantially parallel gas flow.

Liquid, including a desired concentration of therapeutic substances, from one or more pressurized therapeutic liquid sources (not shown) enters device 100 through liquid inlet port 110 (Fig. 2) and passes, as indicated by arrow 132, through liquid communication tube 118 (Figs. 2 and 4). In turn, at distal end 106, therapeutic liquid is discharged through an opening referenced 128 in the distal end of liquid discharge nozzle 116 into the discharging flow 126 of gas, the therapeutic liquid flow being indicated by arrow 130.

It will be appreciated by persons skilled in the art that, as the pressurized discharging gas emerges 126 from gas discharge nozzle 114 into the atmosphere, it undergoes a rapid drop in pressure to atmospheric pressure. The sudden pressure drop results in a substantial acceleration of the velocity of the discharging gas flow that approximates or even exceeds the velocity of sound and results in the production of a shock wave. The effect of the shock wave is to atomize the therapeutic liquid discharging from liquid discharge nozzle 116 into the flow of gas as a stream of therapeutic liquid droplets 130, such that there is obtained a relatively narrow jet of therapeutic liquid droplets in a high velocity gas flow 126.

Further, by way of example, the proportion of liquid flow to gas flow is extremely low due to the relatively high gas pressure of about 685 KPa (100 psi) and low liquid pressure of about 14 KPa (2 psi), as well as the relatively large internal diameter of gas discharge nozzle 114 (about 0.5 mm) compared to a small internal diameter (about 0.09 mm) of liquid discharge nozzle 116. Consequently, little liquid tends to accumulate at the site to be cleaned or treated. Furthermore, the relatively high gas flow has the effect of dispersing any accumulated liquid. When using a jet utilizing only liquid for cleansing, the liquid tends to accumulate on the tissue surface resulting in formation of a virtually stagnant liquid boundary layer close to and in contact with the surface, thereby reducing the effectiveness of cleansing. The very thin to negligible layer of liquid produced on the tissue surface by the present invention allows more efficient dosage of additional therapeutic substances to the tissue surface, including the possibility of subcutaneous application of the therapeutic substances, as discussed hereinabove.

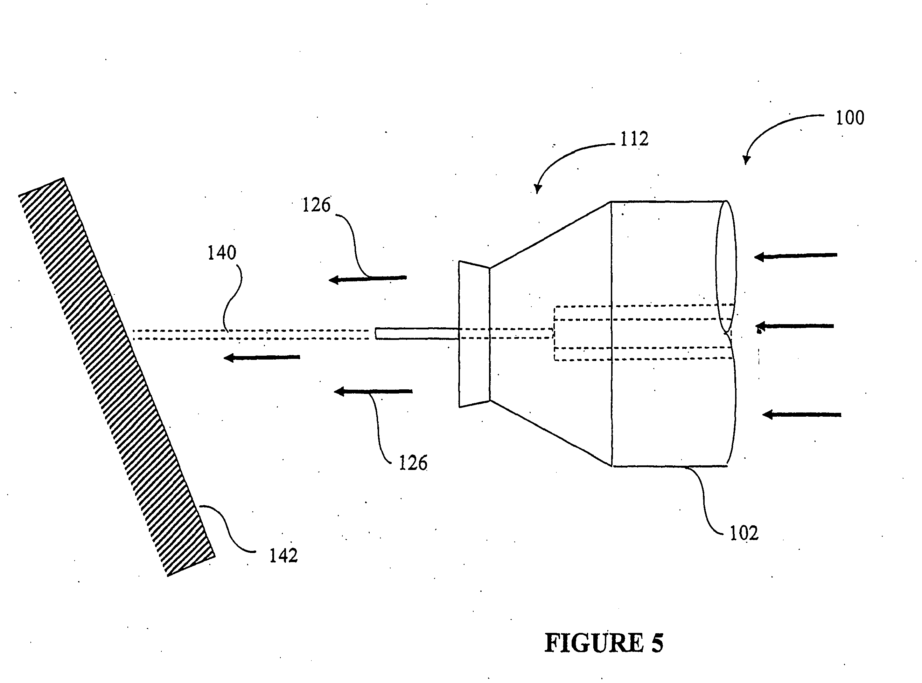

Referring now to Figure 5, there is seen a high velocity flow of therapeutic liquid droplets referenced 140 discharging, in a high velocity gas flow 126, from nozzle arrangement 112 against a tissue surface referenced 142 to be cleaned or treated. Device 100 is held in the hand of a user by housing portion 102.

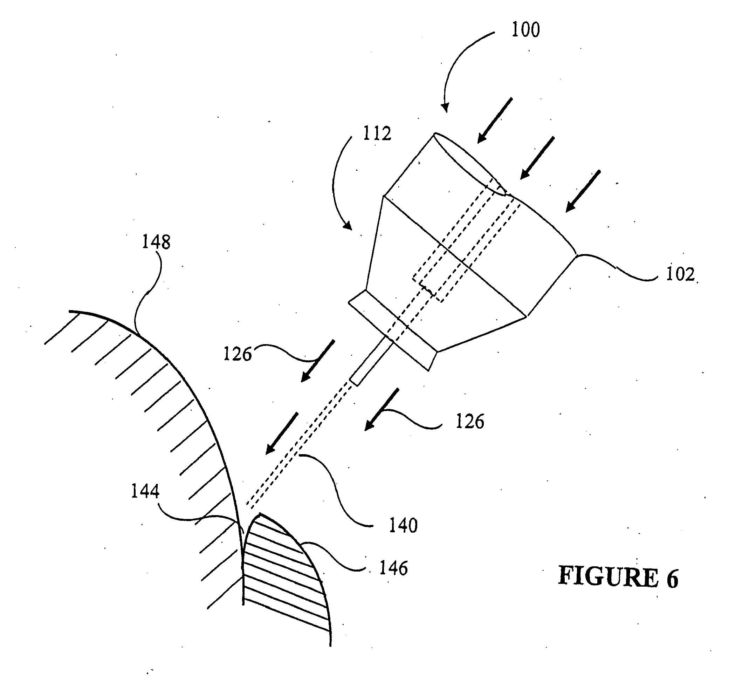

Referring now to Figure 6, there is seen a flow of therapeutic liquid droplets 140 discharging, in a high velocity gas flow 126, from nozzle arrangement 112 of device 100 into a periodontal pocket referenced 144 disposed between a gum referenced 146 and a tooth wall referenced 148. Device 100 is held in the hand of a user by housing portion 102. This procedure is especially effective for cleansing periodontal pockets, subsequent to a dental descaling treatment, so as to remove plaque and calculus debris as well as bacteria and the toxins produced by the bacteria, which otherwise lead to mechanical irritation and inflammation of the gingiva. Device 100 can further be used to apply desired dental therapeutic substances, such as antibiotics or anesthetics to the dental pocket.

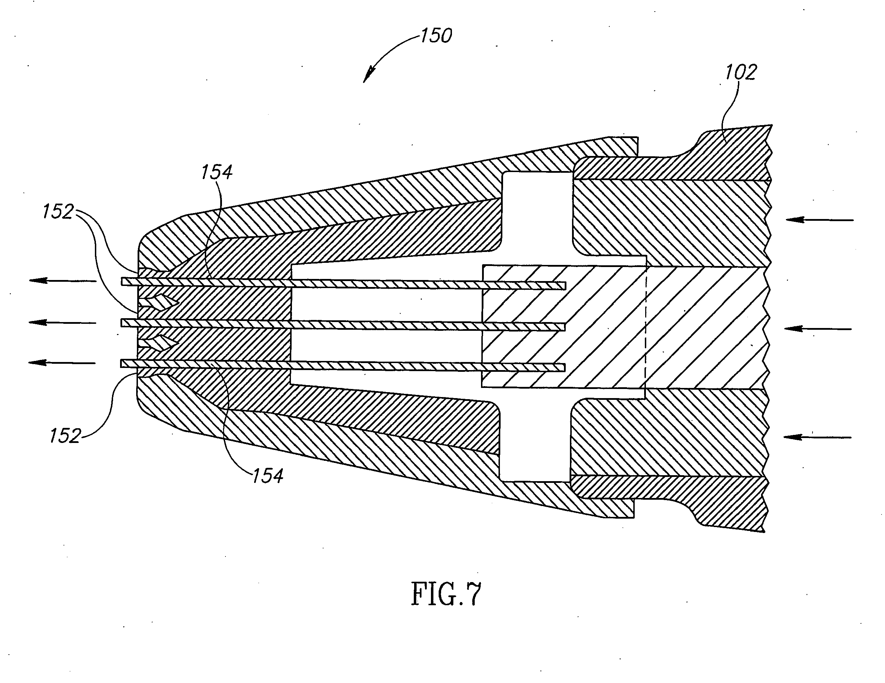

Referring now to Figure 7, there is seen, according to an alternative embodiment of the present invention, a cross-sectional view of a device (not shown) having a housing portion 102 and a multiple nozzle arrangement referenced generally 150. Nozzle arrangement 150 is configured having multiple gas discharge nozzles referenced 152 and multiple therapeutic liquid discharge nozzles referenced 154 disposed generally concentrically within each gas nozzle 152 and projecting there-beyond. The therapeutic discharge nozzles project past the gas discharge nozzles in Figures 2-6 as well. Such a multiple nozzle arrangement 150 facilitates expanding the rate of tissue cleaning, in the event that the system is used for this purpose. Additionally, the present configuration supports multiple therapeutic liquid flows, which may be individually controlled, as described hereinabove.

It will be appreciated by persons skilled in the art that the present invention is not limited by the drawings and description hereinabove presented. Rather, the invention is defined by the claims that follow.

## Claims

1. A system (500) for administering a therapeutic substance to tissue, which includes:
a) a pressurized gas source (520);
b) a pressurized liquid source (530);
c) at least one pressurized therapeutic liquid source (107, 540); and
d) a device (100, 510) which includes:
i) a gas inlet port (108) connected to said pressurized gas source (520);
ii) a liquid inlet port (110) through which a pressurized liquid moves from said pressurized liquid source (530);
iii) at least one therapeutic liquid inlet port (109), each port (109) connected to at least one of said at least one pressurized therapeutic liquid source (107, 540) controllable by a control device (105) so that said at least one pressurized therapeutic liquid source (107, 540) is operative to supply therapeutic liquid at preselected times for preselected intervals, thereby to control the timing and dosage of the therapeutic liquid; and
iv) a stream jet delivery nozzle (112) arrangement including;
1) at least one gas discharge nozzle (114, 152) arranged to receive a flow of pressurized gas from said gas inlet port (108) and configured to accelerate the flow of gas so as to discharge it at an elevated velocity; and
2) at least one liquid discharge nozzle (116, 154) arranged to receive a flow of therapeutic liquid and operative to discharge the flow of therapeutic liquid into the elevated velocity flow of gas, thereby to similarly accelerate the velocity of the discharged therapeutic liquid as a therapeutic stream of accelerated therapeutic droplets and to discharge said stream of accelerated therapeutic droplets towards a tissue mass desired for therapeutic treatment by said therapeutic droplets.

2. A system (500) according to claim 1, wherein the tissue mass desired for therapeutic treatment is a human scalp.

3. A system (500) according to claim 1 or 2, wherein said at least one therapeutic liquid includes a liquid selected from a group which consists of:
saline solution, and a solution comprising saline solution and at least one additional therapeutic substance and wherein said therapeutic substance is a substance selected from a group which consists of: a medication, a nutrient, and a moisturizer.

4. A system (500) according to any preceding claim, wherein the gas discharged from said at least one gas discharge nozzle (114, 152) is accelerated to a velocity in the range of sub-sonic to supersonic velocity.

5. A system (500) according to any preceding claim, wherein said stream jet delivery nozzle (112) arrangement includes at least two gas discharge nozzles (114, 152).

6. A system (500) according to any preceding claim, wherein said stream jet delivery nozzle (112) arrangement includes at least two liquid discharge nozzles (154).

7. A system (500) according to any preceding claim, wherein said at least one liquid discharge nozzle (116, 154) is disposed substantially concentric and within said at least one gas discharge nozzle (114, 152).

8. A system (500) according to any preceding claim, wherein said at least one gas discharge nozzle (114, 152) is a device configured to have a converging portion (120), a throat portion (122) and a diverging portion (124).

9. A system (500) according to any preceding claim, wherein the flow of gas entering said at least one gas discharge nozzle (114, 152) is at a pressure of a first magnitude, and said at least one gas discharge nozzle (114, 152) is operative to cause a pressure drop in the gas flow therethrough such that the pressure of the gas discharged from said at least one gas discharge nozzle (114, 152) is of a second magnitude, wherein the first magnitude is at least twice the second magnitude, so as to cause a shock wave in the gas and the at least one flow of liquid downstream of said at least one gas discharge nozzle (114, 152) and said at least one liquid discharge nozzle (116, 154) so as to cause atomizing of the therapeutic liquid discharged from said at least one liquid discharge nozzle (116, 154) into a high velocity stream of therapeutic droplets (130, 550), thereby to form a stream of therapeutic droplets suspended in the flow of discharged high velocity gas.

10. A system (500) according to any preceding claim, wherein the therapeutic liquid enters said at least one liquid discharge nozzle (116, 154) via said liquid inlet port (110) after entering said liquid inlet port (110) through said therapeutic liquid inlet port (109).

11. A system (500) according to any one of claims 1 to 9, wherein the therapeutic liquid enters said at least one liquid discharge nozzle (116, 154) directly from said pressurized therapeutic liquid source (107, 540).

12. A system (500) according to any one of claims 1 to 10, wherein the therapeutic liquid in said pressurized therapeutic liquid source (107, 540) is a soluble powder substance soluble in the liquid passing through said liquid inlet port (110) from said pressurized liquid source (530) to said stream jet delivery nozzle (112).

13. A system (500) according to any preceding claim, wherein said control device (105) is a plurality of fluid flow mechanisms which produce a plurality of therapeutic liquid flows.

## Patentansprüche

1. System (500) zum Verabreichen einer therapeutischen Substanz an Gewebe, welches aufweist:
a) eine Druckgasquelle (520);
b) eine Druckflüssigkeitsquelle (530);
c) mindestens eine Quelle (107, 540) für therapeutische Druckflüssigkeit; und
d) eine Vorrichtung (100, 510), welche aufweist:
i) einen mit der Druckgasquelle (520) verbundenen Gaseinlassport (108);
ii) einen Flüssigkeitseinlassport (110), durch welchen sich eine Druckflüssigkeit von der Druckflüssigkeitsquelle (530) aus bewegt;
iii) mindestens einen Einlassport (109) für therapeutische Flüssigkeit, wobei jeder Port (109) mit mindestens einer der mindestens einen Quelle (107, 540) für therapeutische Druckflüssigkeit verbunden ist, welche durch eine Steuervorrichtung (105) derart steuerbar ist, dass die mindestens eine Quelle (107, 540) für therapeutische Druckflüssigkeit derart funktioniert, dass sie therapeutische Flüssigkeit zu vorgewählten Zeitpunkten über vorgewählte Intervalle zuführt, um so die Zeitgebung und die Dosierung der therapeutischen Flüssigkeit zu steuern; und
iv) eine Anordnung von Strahlstromausgabedüsen (112) mit:
1) mindestens einer Gasauslassdüse (114, 152), welche derart angeordnet ist, dass sie einen Druckgasstrom von dem Gaseinlassport (108) aufnimmt und derart funktioniert, dass sie den Gasstrom beschleunigt, so dass sie diesen mit einer hohen Geschwindigkeit ausgibt; und
2) mindestens einer Flüssigkeitsauslassdüse (116, 154), die derart angeordnet ist, dass sie einen Strom therapeutischer Flüssigkeit aufnimmt und derart funktioniert, dass sie den Strom therapeutischer Flüssigkeit in den eine hohe Geschwindigkeit aufweisenden Gasstrom ausgibt, um so in ähnlicher Weise die Geschwindigkeit der ausgegebenen therapeutischen Flüssigkeit in Form eines therapeutischen Stroms beschleunigter therapeutischer Tröpfchen zu beschieunigen und den Strom beschleunigter therapeutischer Tröpfchen in Richtung einer Gewebemasse auszugeben, welche durch die therapeutischen Tropfchen therapeutisch behandelt werden soll.

2. System (500) nach Anspruch 1, bei dem die für die therapeutische Behandlung vorgesehene Gewebemasse die menschliche Kopfhaut ist.

3. System (500) nach Anspruch 1 oder 2, bei dem die mindestens eine therapeutische Flüssigkeit eine Flüssigkeit enthält, welche aus der Gruppe gewählt ist, die aus Salzlösung und einer Lösung, die Salzlösung und mindestens eine zusätzliche therapeutische Substanz enthäit, besteht, und wobei die therapeutische Substanz eine Substanz ist, welche aus einer Gruppe gewählt ist, welche aus einem Medikament, einem Nährstoff und einem Feuchtigkeitsmittel besteht.

4. System (500) nach einem der vorhergehenden Ansprüche, bei den das aus der mindestens einen Gasauslassdüse (114, 152) ausgelassenen Gas auf eine Geschwindigkeit im Bereich zwischen Unterschallgeschwindigkeit und Überschallgeschwindigkeit beschleunigt wird.

5. System (500) nach einem der vorhergehenden Ansprüche, bei dem die Strahlstromausgabedüsenanordnung (112) mindestens zwei Gasauslassdüsen (114, 152) aufweist.

6. System (500) nach einem der vorhergehenden Ansprüche, bei dem die Strahlstromausgabedüsenanordnung (112) mindestens zwei Flüssigkeitsauslassdüsen (154) aufweist.

7. System (500) nach einem der vorhergehenden Ansprüche, bei dem die mindestens eine Flüssigkeitsauslassdüse (116, 154) im Wesentlichen konzentrisch mit und innerhalb der mindestens einen Gasauslassdüse (114, 152) angeordnet ist.

8. System (500) nach einem der vorhergehenden Ansprüche, bei dem die mindestens eine Gasauslassdüse (114, 152) eine Vorrichtung ist, die derart konfiguriert ist, dass einen konvergierenden Bereich (120), einen Halsbereich (122) und einen divergierenden Bereich (124) aufweist.

9. System (500) nach einem der vorhergehenden Ansprüche, bei dem der in die mindestens eine Gasauslassdüse (114, 152) eintretende Gasstrom einen Druck mit einer ersten Höhe aufweist, und die mindestes eine Gasauslassdüse (114, 152) betreibbar ist, um einen Druckabfall in dem hindurchgehenden Gasstrom zu bewirken, so dass der Druck des aus der mindestens einen Gasauslassdüse (114, 154) ausgelassenen Gases eine zweite Höhe aufweist, wobei die erste Höhe mindestens das Doppelte der zweiten Höhe beträgt, um so eine Stoßwelle in dem Gas und dem mindestens einen Flüssigkeitsstrom stromabwärts der mindestens einen Flüssigkeitsauslassdüse (114, 152) und der mindestens einen Flüssigkeitsauslassdüse (116, 154) zu erzeugen, so dass die aus der mindestens einen Flüssigkeitsauslassdüse (116, 154) ausgelassene therapeutische Flüssigkeit zu einem Hochgeschwindigkeitsstrom therapeutischer Tröpfchen (130, 550) zerstäubt wird, um auf diese Weise einen in dem ausgelassenen Hochgeschwindigkeitsgasstrom suspendieren Strom therapeutischer Tröpfchen zu bilden.

10. System (500) nach einem der vorhergehenden Ansprüche, bei dem die therapeutische Flüssigkeit in die mindestens eine Flüssigkeitsauslassdüse (116, 154) über den Flüssigkeitseinlassport (110) eintritt, nachdem sie durch den Einlassport (109) für therapeutische Flüssigkeit in den Flüssigkeitseinlassport (110) eingetreten ist.

11. System (500) nach einem der Ansprüche 1 bis 9, bei dem die therapeutische Flüssigkeit direkt von der Druckquelle (107, 540) für therapeutische Flüssigkeit aus in die mindestens eine Flüssigkeitsauslassdüse (116, 154) eintritt.

12. System (500) nach einem der Ansprüche 1 bis 10, bei dem die therapeutische Flüssigkeit in der Druckquelle (107, 540) für therapeutische Flüssigkeit eine lösliche Pulversubstanz ist, welche in der durch den Flüssigkeitseinlassport (110) von der Druckflüssigkeitsquelle (530) zu der Stromstrahlausgabedüse (112) strömenden Flüssigkeit löslich ist.

13. System (500) nach einem der vorhergehenden Ansprüche, bei dem die Steuervorrichtung (105) aus mehreren Fluidströmungsmechanismen beeteht, welche mehrere Ströme therapeutischer Flüssigkeit erzeugen.

## Revendications

1. Un système (500) permettant d'administrer une substance thérapeutique dans les tissus, qui comprend :
a) une source de gaz pressurisé (520)
b) une source de liquide pressurisé (530)
c) au moins une source de liquide thérapeutique pressurisé (107, 540) ; et
d) un dispositif (100, 510) qui comprend :
i) un orifice d'alimentation de gaz (108) relié à ladite source de gaz pressurisé (520)
ii) un orifice d'alimentation du liquide (110) à travers lequel un liquide pressurisé progresse de ladite source de liquide pressurisé (530);
iii) au moins un orifice d'alimentation du liquide thérapeutique (109), chaque orifice (109) étant relié à au moins une des dites sources de liquide thérapeutique pressurisé (107, 540) contrôlable à l'aide d'un dispositif de contrôle (105) de façon à ce que ladite au moins source de liquide thérapeutique pressurisé (107, 540) puisse fournir le liquide thérapeutique à des heures présélectionnées à intervalles présélectionnés, permettant ainsi de contrôler les séquences et le dosage du liquide thérapeutique ; et
iv) un dispositif de tubulure de livraison type "stream jet" (112) comprenant :
1) au moins une tubulure de décharge de gaz (114, 152) disposée pour recevoir un flux de gaz pressurisé dudit orifice d'alimentation de gaz (108) et configurée pour accélérer le débit de gaz afin de le décharger à vitesse accélérée ; et
2) au moins une tubulure de livraison de liquide (116, 154) disposée de façon à recevoir un flux de liquide thérapeutique et pouvant envoyer le flux de liquide thérapeutique dans le débit de gaz à haute vitesse accélérant ainsi de façon similaire la vitesse du liquide thérapeutique déchargé comme un flot thérapeutique accéléré de gouttelettes thérapeutiques et décharger ledit flot accéléré de gouttelettes thérapeutiques vers une masse tissulaire nécessitant un traitement thérapeutique à l'aide des gouttelettes thérapeutiques.

2. Un système (500) selon la revendication 1, dans lequel la masse tissulaire nécessitant un traitement thérapeutique est un cuir chevelu humain.

3. Un système (500) selon la revendication 1 ou 2, dans lequel ledit liquide thérapeutique comprend un liquide sélectionné dans un groupe qui consiste en une solution saline, et une solution comprenant une solution saline et au moins une substance thérapeutique supplémentaire et dans lequel ladite substance thérapeutique est une substance sélectionnée dans un groupe qui comprend un médicament, un élément nutritif, et un élément hydratant.

4. Un système (500) selon une quelconque des revendications précédentes dans lequel le gaz déchargé d'au moins une dite tubulure de décharge de gaz (114, 152) est accéléré à une vitesse se situant dans la fourchette vélocité sous-sonique et supersonique.

5. Un système (500) selon l'une quelconque des revendications précédentes dans lequel ledit dispositif de tubulure de livraison type "stream jet" (112) comprend au moins deux tubulures de décharge de gaz (114, 152).

6. Un système (500) selon l'une quelconque des revendications précédentes, dans lequel ladite tubulure de livraison type "stream jet" (112) comprend au moins deux tubulures de décharge de liquide (154).

7. Un système (500) selon une quelconque des revendications précédentes dans lequel au moins une tubulure de décharge de liquide (116, 154) est disposée de façon concentrique et à l'intérieur de ladite tubulure de décharge de gaz (114, 152).

8. Un système (500) selon l'une quelconque des revendication précédentes dans lequel au moins une tubulure de décharge de gaz (114, 152) est un dispositif configuré de façon à présenter une partie convergente (120), un étranglement (122) et une partie divergente (124).

9. Un système (500) selon l'une quelconque des revendications précédentes, dans lequel le flux de gaz entrant dans au moins une tubulure de décharge de gaz (114, 152) est à une pression de première grandeur, et au moins une tubulure de décharge de gaz (114, 152) permet d'entraîner une chute de pression dans le flux de gaz telle que la pression du gaz déchargé par au moins une tubulure de décharge (114, 152) est de seconde grandeur, dans lequel la première grandeur est au moins égale au double de la 2^{ème} grandeur, afin de provoquer une onde de choc dans le gaz et au moins un flux de liquide en aval d'au moins une tubulure de décharge de gaz (114, 152) et au moins une tubulure de décharge de liquide (116, 154) afin de provoquer l'atomisation du liquide thérapeutique déchargé à partir d'au moins une tubulure de décharge de liquide (116, 154) en un écoulement à haute vélocité de gouttelettes thérapeutiques (130, 550) formant ainsi un écoulement de gouttelettes thérapeutiques suspendues dans le flux de gaz déchargé à haute vélocité.

10. Un système (500) selon l'une quelconque des revendications précédentes, dans lequel le liquide thérapeutique pénètre dans au moins une tubulure de décharge de liquide (116, 154) via ledit orifice d'alimentation du liquide (110) après avoir pénétré dans l'orifice d'alimentation du liquide (110) à travers ledit orifice d'alimentation du liquide thérapeutique (109).

11. Un système (500) selon l'une quelconque des revendications 1 à 9, dans lequel le liquide thérapeutique pénètre dans au moins une tubulure de décharge de liquide (116, 154) directement à partir de ladite source de liquide thérapeutique pressurisé (107, 540).

12. Un système (500) selon l'une quelconque des revendications 1 à 10, dans lequel le liquide thérapeutique se trouvant dans la source de liquide thérapeutique pressurisé (107, 540) est une poudre soluble dans le liquide qui passe à travers ledit orifice d'alimentation du liquide (110) provenant de ladite source de liquide pressurisé (530) vers ladite tubulure de livraison type "stream jet" (112).

13. Un système (500) selon l'une quelconque des revendications précédentes, dans lequel ledit dispositif de contrôle (105) est constitué d'une pluralité de mécanismes de débit de fluide qui produit une pluralité d'écoulements de liquide thérapeutique.
